# EUROPÄISCHE PATENTSCHRIFT

(11) **EP 2 794 016 B1**
(45) Veröffentlichungstag und Bekanntmachung des Hinweises auf die Patenterteilung: **04.04.2018**
(21) Anmeldenummer: 12786982.4
(22) Anmeldetag: 13.11.2012
(51) Int. Cl.: A61Q 5/06, A61K 8/35, A61K 8/37

(54) **MITTEL ZUM FÄRBEN VON KERATINISCHEN FASERN ENTHALTEND KATIONISCHE ANTHRACHINONFARBSTOFFE UND FETTSÄUREDIESTER VON ALKANDIOLEN**
SUBSTANCE FOR DYEING KERATIN FIBERS, CONTAINING CATIONIC ANTHRAQUINONE DYES AND FATTY ACID DIESTERS OF ALKANE DIOLS
AGENT DE COLORATION DE FIBRES KÉRATINIQUES CONTENANT DES COLORANTS ANTHRAQUINONIQUES CATIONIQUES ET DES DIESTERS D'ACIDE GRAS D'ALCANE-DIOL

(30) Priorität: 20.12.2011 DE 102011089222
(43) Veröffentlichungstag der Anmeldung: 29.10.2014
(73) Patentinhaber: Henkel AG & Co. KGaA, 40589 Düsseldorf (DE)
(72) Erfinder: WESER, Gabriele, 41472 Neuss (DE); KOLONKO, Claudia, 42857 Remscheid (DE)
(86) Internationale Anmeldenummer: PCT/EP2012/072477
(87) Internationale Veröffentlichungsnummer: WO 2013/092007

(56) Entgegenhaltungen:
- EP-A1- 1 820 826
- EP-A1- 2 272 492
- WO-A2-01/76552
- CA-A1- 2 613 049
- DE-A1-102008 036 957
- US-A- 5 520 707

## Beschreibung

Die vorliegende Erfindung betrifft Mittel zum Färben und gegebenenfalls gleichzeitigen Aufhellen von keratinischen Fasern, insbesondere menschlichen Haaren, welche kationische Anthrachinonfarbstoffe und spezielle Fettsäurediester von Alkandiolen enthalten. Weiterhin betrifft die Erfindung die Verwendung dieser Mittel zur Erzeugung von Haarfärbungen mit erhöhtem Glanz, intensivem Farbergebnis, verbesserten Echtheitseigenschaften und verringerter Selektivität.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken. Direktziehende Farbstoffe werden unter schonenderen Bedingungen appliziert. Ihr Nachteil liegt jedoch darin, dass die Färbungen häufig nur über unzureichende Echtheitseigenschaften verfügen, insbesondere bei der Haarwäsche, aber auch gegenüber äußeren Einflüssen, wie Sonnenlicht oder reaktiven Umweltchemikalien, wie beispielsweise Schwimmbadwasser.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Eine besondere Herausforderung für die Haarfärbung mit direktziehenden Farbstoffen stellt die gleichmäßige Färbung von häufig vorbehandeltem Haar, wie beispielsweise gebleichtem oder dauergewelltem Haar, dar, bei dem die Faser in den verschiedenen Längen oder verschieden behandelten Bereichen eine unterschiedlich starke Vorschädigung besitzt. Während der Färbung selbst kann das Färbemittel auf unterschiedlich vorgeschädigtem Haar ein ungleichmäßiges Färbeverhalten zeigen, aber auch durch wiederholte Haarwäschen können die Farbstoffe in den unterschiedlichen Haarbereichen verschieden stark auswaschen werden, wodurch ein uneinheitliches und damit unerwünschtes Farbergebnis erzielt wird.

Die Herstellung von Färbeformulierungen mit verringerter Selektivität, mittels welcher auf den unterschiedlich stark geschädigten Partien der Haare ein gleichmäßiges Farbergebnis erzielt werden kann, steht bei der Entwicklung Färbeprodukten auf Basis von Direktziehern nach wie vor besonders im Fokus. Insbesondere soll diese verringerte Selektivität nicht nur direkt nach dem Färbeprozeß, sondern auch nach wiederholten Haarwäschen noch vorhanden sein.

Zum Färben von keratinischen Fasern werden oft tensidhaltige Formulierungen eingesetzt. Der Einsatz von Tensiden ist mit dem Nachteil behaftet, dass während des Haarfärbeprozesses eine zu starke Entfettung der Kopfhaut auftreten kann, welche zu juckender Kopfhaut oder schlimmstenfalls zu Schuppenbildung führt. Zum Schutz der Kopfhaut können in den Haarfärbeformulierungen rückfettende Substanzen eingesetzt werden, welche den Lipidgehalt der Kopfhaut wieder ausgleichen.

Rückfettende Substanzen mit positiver Wirkung auf der Kopfhaut können jedoch auf der Haarfaser einen negative Wirkung entfalten, da das Haar durch die rückfettenden Substanzen zu schnell fettig erscheint, was die Frisur ungepflegt und unattraktiv wirken läßt. Trotz der Vielzahl der inzwischen aus dem Stand der Technik bekannten Wirkstoffe besteht daher nach wie vor immer noch Bedarf an Substanzen- bzw. Substanzkombinationen, welche die Kopfhaut beim Färbevorgang schützen und dem Haar Glanz verleihen, ohne es hierbei aber vorschnell fettig erscheinen zu lassen.

Zusätzlich kann sich beim Einsatz von manchen Wirkstoffen herausstellen, dass diese mit den verwendeten Farbstoffen nicht kompatibel sind, so dass das bei Kombination von Wirk- und Farbstoff erhaltene Farbergebnis im Vergleich zum Einsatz des Farbstoffes allein schlechter ist. Daher besteht insbesondere Bedarf an geeigneten Kombinationen aus Wirk- und Farbstoff, bei welchen sich der positive Effekt des Wirkstoffes entfalten kann, ohne sich gleichzeitig nachteilig auf die Echtheitseigenschaften des Farbstoffes auszuwirken.

Die Aufgabe der vorliegenden Erfindung ist daher die Bereitstellung eines Färbemittels für keratinische Fasern, insbesondere menschliche Haare, welches neben anderen positiven Echtheitseigenschaften insbesondere eine geringe Selektivität (bzw. ein gutes Egalisiervermögen) und eine gute Waschechtheit besitzt. Gleichzeitig soll die Kopfhaut während des Färbevorgangs geschützt und den keratinischen Fasern Glanz verliehen werden, ohne sie hierbei jedoch vorschnell fettig erscheinen zu lassen.

Der Einsatz von kationischen Anthrachinonfarbstoffen in Produkten zum Färben von keratinischen Fasern ist prinzipiell bereits aus dem Stand der Technik, beispielsweise aus EP 1 006 154 B1 oder aus EP 1 820 826 A1, bekannt. Ferner werden in EP 2 329 809 Kombinationen von kationischen Anthrachinonfarbstoffen mit Oxidationsfarbstoffvorprodukten vom Entwicklertyp für die oxidative Färbung von Haaren beansprucht.

Die Kombinationen der kationischen Anthrachinone mit speziellen Fettsäurediestern eines Alkandiols sind im Stand der Technik bislang noch nicht beschrieben. Im Zuge der zu dieser Erfindung führenden Arbeiten hat sich nun in überraschender Weise gezeigt, dass diese Kombinationen zu Färbungen führen, welche die oben beschriebene Aufgabenstellung in herausragendem Maß erfüllen.

Ein erster Gegenstand der vorliegenden Erfindung ist ein Mittel zum Färben und gegebenenfalls gleichzeitigen Aufhellen von keratinischen Fasern, insbesondere menschlichen Haaren, welches in einem kosmetischen Träger
(a) mindestens eine Verbindung der Formel (I) 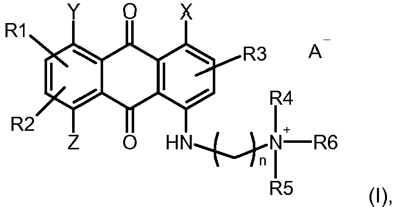 worin
   - R1, R2, R3: jeweils unabhängig voneinander für Wasserstoff, Halogen, eine Nitrogruppe, eine Cyanogruppe, eine Carboxylgruppe, eine Sulfonsäuregruppe, eine Hydroxygruppe, eine C₁-C₆-Acylaminogruppe, eine Carboxamidgruppe, eine Sulfonamidgruppe, eine C₁-C₆-Alkylgruppe, C₁-C₆-Alkoxygruppe, eine C₂-C₆-Alkenylgruppe, eine C₂-C₆-Hydroxyalkylgruppe oder eine C₁-C₆-Alkoxy-C₂-C₆-alkylgruppe stehen;
   - R4, R5, R6: jeweils unabhängig voneinander für eine C₁-C₆-Alkylgruppe, eine C₂-C₆-Alkenylgruppe, für eine C₂-C₆-Hydroxyalkylgruppe oder eine C₁-C₆-Alkoxy-C₂-C₆-alkylgruppe stehen;
   oder R4 und R5 bilden zusammen mit dem quartären Stickstoffatom, an das sie gebunden sind, einen 5-, 6- oder 7-gliedrigen Ring, der gegebenenfalls weitere Heteroatome enthalten kann und/oder weitere Substituenten tragen kann;
   - X, Y, Z: jeweils unabhängig voneinander für Wasserstoff, eine Hydroxygruppe oder eine Gruppe N(R7)(R8) stehen,
   worin
   R7 und R8 jeweils unabhängig voneinander für Wasserstoff, eine C₁-C₆-Alkylgruppe, eine C₂-C₆-Alkenylgruppe, für eine C₂-C₆-Hydroxyalkylgruppe oder eine C₁-C₆-Alkoxy-C₂-C₆-alkylgruppe stehen,
   unter der Maßgabe, dass mindestens einer der Reste X, Y, Z für eine Gruppe N(R7)(R8) steht,
   - n: für eine Zahl von 2 bis 6 steht,
   - A⁻: für ein physiologisch verträgliches Anion steht,
   und
(b) mindestens einen Fettsäurediester eines Alkandiols enthält,
wobei das Mittel den oder die Fettsäurediester eines Alkandiols (b) in einem Anteil von 0,5 bis 5,0 Gew.-%, ieweils bezogen auf das Gesamtgewicht des Mittels, enthält. Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Der erfindungsgemäß verwendete Begriff "Färben von Keratinfasern" umfasst jedwede Form der Farbveränderung der Fasern. Umfasst sind insbesondere die unter den Begriffen Tönung, Aufhellung, Blondierung, Bleiche, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen.

Die erfindungsgemäßen Mittel enthalten das bzw. die kationischen Anthrachinone der Formel (I) und das bzw. die Fettsäurediester von Alkandiolen in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Es ist aber auch denkbar, die erfindungsgemäßen Mittel in eine pulverförmige oder auch tablettenförmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Als ersten wesentlichen Inhaltsstoff (a) enthalten die erfindungsgemäßen Mittel mindestens einen direktziehenden kationischen Anthrachinonfarbstoff der allgemeinen Formel (I).

Die Substituenten R1 bis R8 der Verbindung der Formel (I) sind nachfolgend beispielhaft erläutert: Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine C₁-C₆-Hydroxyalkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Erfindungsgemäß bevorzugte C₁-C₆-Alkoxygruppen sind die Methoxy- oder die Ethoxygruppe. Beispiele für Halogenatome sind F-, Cl-, Br- oder I-Atome, wobei Br- oder Cl-Atome ganz besonders bevorzugt sind. Bevorzugte Beispiele für erfindungsgemäße C₁-C₆-Alkoxy-C₂-C₆-alkylgruppen sind die Methoxyethyl-, Ethoxyethyl-, Methoxypropyl-, Methoxybutyl-, Ethoxypropyl-, Ethoxybutyl- und die Methoxyhexylgruppe. Beispiele für eine C₁-C₆-Acylaminogruppe sind die Acetamidgruppe, die Propanamidgruppe und die Butanamidogruppe, wobei die Acetamidgruppe bevorzugt ist. Als bevorzugte Beispiele für einen 5-, 6- oder 7-gliedrigen Ring, der aus R4, R5 und dem quartären Stickstoffatom gebildet wird, sind der Pyrrolidiniumring, der Piperidiniumring, der Morpholiniumring und der 1-Azepaniumring zu nennen.

Farbstoffe der Formel (I), in denen R1, R2 und R3 unabhängig voneinander für Wasserstoff, Halogen, eine Carboxylgruppe, eine Sulfonsäuregruppe, eine C₁-C₆-Alkylgruppe oder eine C₁-C₆-Alkoxygruppe stehen, liefern besonders intensive Färbeergebnisse und sind daher bevorzugt.

Weiterhin ist es bevorzugt, wenn einer der Reste ausgewählt aus R1, R2 und R3 für Halogen, eine Carboxylgruppe, eine Sulfonsäuregruppe, eine C₁-C₆-Alkylgruppe oder eine C₁-C₆-Alkoxygruppe steht und die anderen beiden Reste ausgewählt aus R1, R2 und R3 beide für Wasserstoff stehen.

Eine bevorzugte Ausführungsform ist ein Mittel zum Färben und gegebenenfalls gleichzeitigen Aufhellen von keratinischen Fasern, welches dadurch gekennzeichnet ist, dass es eine Verbindung der Formel (I) enthält, worin wenigstens einer der Reste R1, R2 und/oder R3 für eine C₁-C₆-Alkylgruppe steht.

Bei besonders geeigneten Verbindungen der Formel (I) steht einer der Reste ausgewählt aus R1, R2 und R3 für eine C₁-C₆-Alkylgruppe und die anderen beiden Reste ausgewählt aus R1, R2 und R3 für Wasserstoff.

In einer explizit ganz besonders bevorzugten Ausführungsform stehen R1 und R2 beide für ein Wasserstoffatom, und R3 steht für eine Methylgruppe.

Des weiteren werden besonders mit den Mitteln gute Färbeergebnisse erzielt, die mindestens eine Verbindung der Formel (I) enthalten, bei welcher die Reste R4, R5 und R6 unabhängig voneinander für eine C₁-C₆-Alkylgruppe oder eine Alkenylgruppe stehen. Insbesondere bevorzugt steht jeder der Reste R4, R5 und R6 für eine C₁-C₆-Alkylgruppe.

Ganz besonders bevorzugt ist es, wenn R4 und R5 beide für eine Methylgruppe stehen und R6 für eine Methylgruppe, eine Ethylgruppe oder eine n-Propylgruppe steht.

Weiterhin ist es ganz besonders bevorzugt, wenn R4 und R5 beide für eine Methylgruppe stehen und R6 für eine n-Propylgruppe steht.

In einer ebenfalls besonders bevorzugten Ausführungsform stehen die Reste R4, R5 und R6 jeweils für eine Methylgruppe.

Bei Verbindungen der Formel (I) besteht die Maßgabe, dass mindestens einer der Reste X, Y, Z für eine Gruppe N(R7)(R8) steht. Es konnten insbesondere dann Färbungen mit guten anwendungstechnischen Eigenschaften erhalten werden, wenn Verbindungen der Formel (I) eingesetzt wurden, bei denen X für eine Gruppe N(R7)(R8) steht und Y und Z jeweils für Wasserstoff stehen.

R7 und R8 stehen bevorzugt unabhängig voneinander für Wasserstoff oder eine C₁-C₆-Alkylgruppe. Besonders bevorzugt stehen R7 und R8 unabhängig voneinander für Wasserstoff oder für eine Methylgruppe. Verbindungen der Formel (I), bei denen sowohl R7 als auch R8 für Wasserstoff stehen, haben sich als besonders geeignet erwiesen und sind daher besonders bevorzugt.

Im Rahmen der zu dieser Erfindung führenden Arbeiten hat es sich als ganz besonders vorteilhaft herausgestellt, wenn X für eine Gruppe NH2 steht.

Eine weitere bevorzugte Ausführungsform ist daher ein Mittel zum Färben und gegebenenfalls gleichzeitigen Aufhellen von keratinischen Fasern, welches dadurch gekennzeichnet ist, dass es eine Verbindung der Formel (I) enthält, worin mindestens X für eine Gruppe NH₂ steht.

n steht bevorzugt für die Zahlen 2 oder 3 und ganz besonders bevorzugt für die Zahl 3.

A⁻ steht für ein physiologisch verträgliches Anion. Geeignete physiologisch verträgliche Anionen sind Halogenid, Hydrogensulfat, ½ Sulfat, Benzolsulfonat, p-Toluolsulfonat, Acetat, Citrat, Lactat, ½ Tartrat, Methylsulfat (H₃COSO₃⁻) oder Trifluormethansulfonat. Besonders bevorzugt steht A⁻ für Bromid oder für Methylsulfat (H₃COSO₃⁻), ganz besonders bevorzugt steht A⁻ für Methylsulfat (H₃COSO₃⁻).

Erfindungsgemäß bevorzugte Mittel zum Färben und gegebenenfalls gleichzeitigen Aufhellen keratinischer Fasern sind dadurch gekennzeichnet, dass sie mindestens eine Verbindung der allgemeinen Formel (I) enthalten, die ausgewählt ist aus
- 3-[(4-Amino-9,10-dihydro-3-methyl-9,10-dioxo-1-anthracenyl)amino]-N,N,N-trimethyl-1-propanaminiummethylsulfat,
- 3-[(4-Amino-9,10-dihydro-3-methyl-9,10-dioxo-1-anthracenyl)amino]-N,N,N-trimethyl-1-propanaminiumbromid,
- 3-[(4-Amino-9,10-dihydro-3-methyl-9,10-dioxo-1-anthracenyl)amino]-N,N,N-trimethyl-1-propanaminiumchlorid,
- 3-[(4-Amino-9,10-dihydro-3-methyl-9,10-dioxo-1-anthracenyl)amino]-N,N,N-trimethyl-1-propanaminium-p-toluolsulfonat,
- 3-[(4-Amino-9,10-dihydro-3-methyl-9,10-dioxo-1-anthracenyl)amino]-N,N,N-trimethyl-1-propanaminiumacetat,
- 3-{[9,10-Dihydro-4-(methylamino)-9,10-dioxo-1-anthracenyl]amino}-N,N-dimethyl-N-propyl-1-propanaminiummethylsulfat,
- 3-{[9,10-Dihydro-4-(methylamino)-9,10-dioxo-1-anthracenyl]amino}-N,N-dimethyl-N-propyl-1-propanaminiumbromid,
- 3-{[9,10-Dihydro-4-(methylamino)-9,10-dioxo-1-anthracenyl]amino}-N,N-dimethyl-N-propyl-1-propanaminiumchlorid,
- 3-{[9,10-Dihydro-4-(methylamino)-9,10-dioxo-1-anthracenyl]amino}-N,N-dimethyl-N-propyl-1-propanaminium-p-toluolsulfonat und
- 3-{[9,10-Dihydro-4-(methylamino)-9,10-dioxo-1-anthracenyl]amino}-N,N-dimethyl-N-propyl-1-propanaminiumacetat.

Als optimal geeignet zur Lösung der erfindungsgemäßen Aufgabenstellung hat sich die Verbindung der Formel (Ia) herausgestellt. 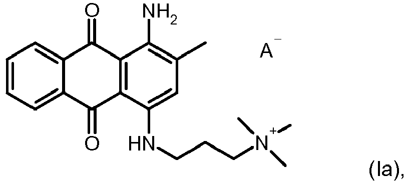 worin A⁻ für ein physiologisch verträgliches Anion, bevorzugt für Methylsulfat (H₃COSO₃⁻), steht.

Eine weitere besonders bevorzugte Ausführungsform ist daher ein Mittel zum Färben und gegebenenfalls gleichzeitigen Aufhellen von keratinischen Fasern, welches dadurch gekennzeichnet ist, dass es als Verbindung der Formel (I) die Verbindung gemäß Formel (Ia) enthält, 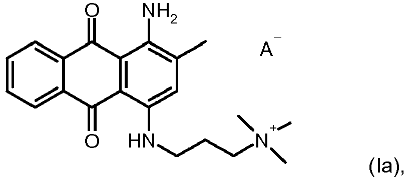 worin A⁻ für ein physiologisch verträgliches Anion, bevorzugt für Methylsulfat (H₃COSO₃⁻), steht.

Die erfindungsgemäßen Mittel zum Färben und gegebenenfalls gleichzeitigen Aufhellen von Keratinfasern enthalten die Verbindung(en) der Formel (I) vorzugsweise in Mengen oberhalb von 0,0001 Gew.-% und unterhalb von 5 Gew.-%, jeweils bezogen auf das gesamte Mittel.

Eine bevorzugte Ausführungsform ist dabei ein Mittel, welches die Verbindung(en) der Formel (I) in einem Anteil von 0,0001 bis 5 Gew.-%, bevorzugt 0,005 bis 3,5 Gew.-%, besonders bevorzugt 0,01 bis 2,5 Gew.-%, insbesondere 0,05 bis 1,5 Gew.-%, und insbesondere bevorzugt von 0,01 bis 1,0 Gew.-%, jeweils bezogen auf das Gesamtgewicht des Mittels, enthält.

Als zweiten wesentlichen Formulierungsbestandteil (b) enthalten die erfindungsgemäßen Mittel mindestens einen Fettsäurediester eines Alkandiols.

Unter einem Fettsäurediester eines Alkandiols wird im Sinne der vorliegenden Erfindung ein Alkandiol verstanden, dessen Hydroxygruppen beide jeweils mit einer Fettsäure verestert sind.

Hierbei kann das Alkandiol sowohl mit zwei gleichen als auch mit zwei unterschiedlichen Fettsäuren Estergruppen ausbilden.

Unter Fettsäuren sind erfindungsgemäß gesättigte oder ungesättigte, unverzweigte oder verzweigte, unsubstituierte oder substituierte C₈-C₂₄-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Unter Alkandiolen werden im Sinne der vorliegenden Erfindungen Alkylketten, insbesondere C₂-C₁₀-Alkylketten, verstanden, die zwei Hydroxygruppen tragen. Die beiden Hydroxygruppen befinden sich an unterschiedlichen Kohlenstoffatomen der C₂-C₁₀-Alkylkette. Neben den zwei Hydroxygruppen können die C₂-C₁₀-Alkylketten noch weitere Substituenten tragen. Es ist jedoch bevorzugt, wenn in den C₂-C₁₀-Alkylketten neben den beiden Hydroxygruppen keine weiteren Substituenten vorhanden sind. Bevorzugt sind 1,2-Alkandiole und 1,3-Alkandiole.

Besonders bevorzugt werden als Fettsäurediester eines Alkandiols (b) mit Fettsäuren verestertes Ethylenglykol und/oder mit Fettsäuren verstertes Propylengklykol in den erfindungsgemäßen Mitteln eingesetzt.

Besonders geeignete Vertreter von Fettsäurediestern eines Alkandiols (b) fallen in die Formel (II), 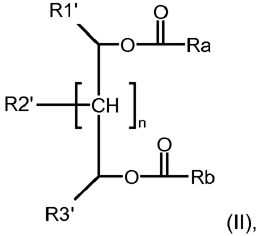 worin
- R1', R3': unabhängig voneinander für Wasserstoff, eine C₁-C₈-Alkylgruppe, eine Arylgruppe oder eine Hydroxy-C₁-C₆-alkylgruppe stehen,
- R2': für Wasserstoff, eine Hydroxygruppe, eine C₁-C₈-Alkylgruppe, eine Arylgruppe oder eine Hydroxy-C₁-C₆-alkylgruppe steht,
- n: für die Zahlen 0 bis 8 steht und
- Ra, Rb: unabhängig voneinander für eine gesättigte oder ungesättigte, unverzweigte oder verzweigte, unsubstituierte oder substituierte C₇-C₂₃-Alkylgruppe stehen.

Bevorzugt stehen R1' und R3' unabhängig voneinander für Wasserstoff, eine C₁-C₈-Alkylgruppe oder eine Hydroxy-C₁-C₆-alkylgruppe, insbesondere bevorzugt für Wasserstoff oder eine Methylgruppe.

Weiterhin ist es bevorzugt, wenn R2' für Wasserstoff, eine Hydroxygruppe oder eine C₁-C₈-Alkylgruppe steht. Insbesondere bevorzugt steht R2 für Wasserstoff.

n steht bevorzugt für 0 oder 1. Besonders bevorzugt steht n für 0.

Geeignete Fettsäurediesters eines Alkandiols (b) zeichnen sich dadurch aus, dass mindestens eine der Estergruppen ausgehend vom Alkandiol mit einer gesättigten Fettsäure ausgebildet wird. Als gesättigte Fettsäure wird in diesem Zusammenhang bevorzugt eine Fettsäure ausgewählt aus

| | Fettsäure | Ra und/oder Rb = |
|---|---|---|
| F1 | Octansäure (Caprylsäure) | -C₇H₁₅ |
| F2 | Decansäure (Caprinsäure) | -C₉H₁₉ |
| F3 | Docdecansäure (Laurinsäure) | -C₁₁H₂₃ |
| F4 | Tetradecansäure (Myristinsäure) | -C₁₃H₂₇ |
| F5 | Hexadecansäure (Palmitinsäure) | -C₁₅H₃₁ |
| F6 | Octadecansäure (Stearinsäure) | -C₁₇H₃₅ |
| F7 | Eicosansäure (Arachinsäure) | -C₁₉H₃₉ |
| F8 | Docosansäure (Behensäure) | -C₂₁H₄₃ |
| F9 | Tetracosansäure (Lignocerinsäure) | -C₂₃H₄₇ |

eingesetzt.

Innerhalb dieser Ausführungsform ist es besonders vorteilhaft, wenn das Mittel einen Fettsäurediester eines Alkandiols (b) enthält, bei dem mindestens eine Esterbindung mit einer gesättigten C₁₄-C₂₀-Fettsäure ausgebildet wird.

Damit sind die Verbindungen der allgemeinen Formel (II) besonders bevorzugt, bei denen mindestens einer der Reste Ra und/oder Rb für eine gesättigte, unverzweigte oder verzeigte, unsubstituierte oder substituierte C₁₃-C₁₉-Alkylgruppe steht.

Ganz besonders geeignet sind Verbindungen der Formel (II), bei denen mindestens einer der Reste ausgewählt aus Ra und/oder Rb für den Rest -C₁₃H₂₇ (F4), -C₁₅H₃₁ (F5), -C₁₇H₃₅ (F6) und/oder -C₁₉H₃₉ (F7) steht.

Des weiteren besitzen auch die Fettsäuretriglyceride eine gute Eignung, bei welchen mindestens eine der Estergruppen ausgehend vom Alkandiol mit einer ein- oder mehrfach ungesättigten Fettsäure ausgebildet wird. Als ungesättigte Fettsäure wird hierbei bevorzugt eine Fettsäure ausgewählt aus

| | Fettsäure | Ra und/oder Rb = |
|---|---|---|
| F11 | Petroselinsäure [(Z)-6-Octadecensäure]. | 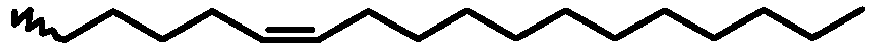 |
| F12 | Palmitoleinsäure [(9Z)-Hexadec-9-ensäure] | 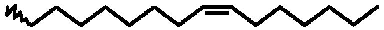 |
| F13 | Ölsäure | 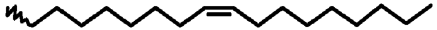 |
| | [(9Z)-Octadec-9-ensäure] | |
| F14 | Elaidinäsure [(9E)-Octadec-9-ensäure] | 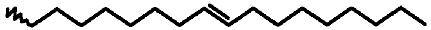 |
| F15 | Erucasäure [(13Z)-Docos-13-ensäure] | 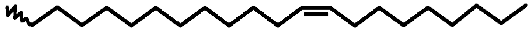 |
| F16 | Linolsäure [(9Z,12Z)-Octadeca-9,12-diensäure | 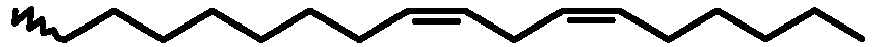 |
| F17 | Linolensäure [(9Z,12Z,15Z)-Octadeca-9,12,15-triensäure | 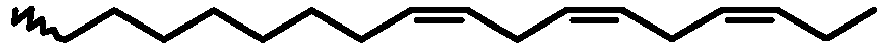 |
| F18 | Elaeostearinäure [(9Z,11E,13E)-Octadeca-9,11,3-triensäure] | 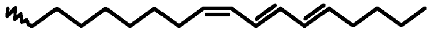 |
| F19 | Arachidonsäure [(5Z,8Z,11Z,14Z)-Icosa-5,8,11,14-tetraensäure] | 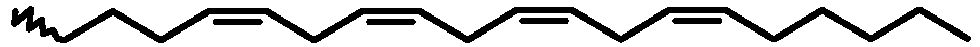 |
| F20 | Nervonsäure [(15Z)-Tetracos-15-ensäure] | 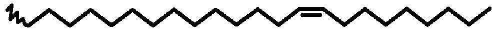 |

eingesetzt.

Im Zuge der zu dieser Erfindung führenden Arbeiten hat es sich weiterhin als bevorzugt herausgestellt, wenn das Färbemittel mindestens einen Fettsäurediester eines Alkandiols (b) enthält, bei dem mindestens eine Esterbindung des Alkandiols mit einer ein-, zwei- oder dreifach ungesättigten C₁₆-C₂₀-Fettsäure ausgebildet wird. Insbesondere ist es bevorzugt, wenn mindestens eine der Fettsäuren ausgewählt aus Palmitoleinsäure (F12), Ölsäure (F13), Linolsäure (F16) und/oder Linolensäure (F17) mit einem Alkandiol verestert sind.

Damit haben sich auch die Verbindungen der Formel (II) als besonders vorteilhaft herausgestellt, bei denen mindestens einer der Reste Ra und/oder Rb für einen der Reste F12, F13, F16 und/oder F17 steht.

Die zur Ausbildung der Ester in den Fettsäurediestern eines Alkandiols (b) eingesetzten Fettsäuren können auch einen oder mehrere Substituenten tragen. Hierbei tragen substituierte Fettsäuren bevorzugt einen oder mehrere Substituenten ausgewählt aus einer Hydroxygruppe, einer Carbonylgruppe und einer C₁-C₆-Alkoxygruppe. Bevorzugt sind die Fettsäuren mit einer Hydroxygruppe oder einer Methoxygruppe substituiert. Als substituierte Fettsäure wird bevorzugt eine Fettsäure ausgewählt aus

| | Fettsäure | Ra und/oder Rb = |
|---|---|---|
| F21 | Ricinolsäure [(12-Hydroxy-(Z)-octade-9-ensäure | 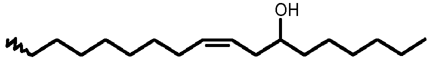 |
| F22 | 12-Hydroxy-octadecansäure | 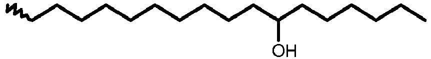 |

eingesetzt.

Verbindungen der Formel (II), bei denen mindestens einer der Reste Ra und/oder Rb für einen der Reste F21 oder F22 steht, sind besonders bevorzugt.

Eine weitere besonders bevorzugte Ausführungsform ist daher ein Mittel zum Färben und gegebenenfalls gleichzeitigen Aufhellen von keratinischen Fasern, welches dadurch gekennzeichnet ist, dass es als Fettsäurediester eines Alkandiols (b) eine Verbindung der Formel (II) enthält, 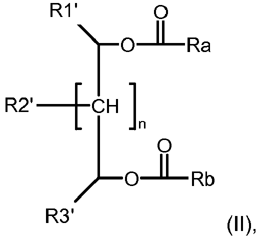 worin
- R1', R3': unabhängig voneinander für Wasserstoff, eine C₁-C₈-Alkylgruppe, eine Arylgruppe oder eine Hydroxy-C₁-C₆-alkylgruppe stehen,
- R2': für Wasserstoff, eine Hydroxygruppe, eine C₁-C₈-Alkylgruppe, eine Arylgruppe oder eine Hydroxy-C₁-C₆-alkylgruppe steht,
- n: für die Zahlen 0 bis 8 steht und
- Ra, Rb: unabhängig voneinander für eine gesättigte oder ungesättigte, unverzweigte oder verzeigte, unsubstituierte oder substituierte C₇-C₂₃-Alkylgruppe stehen, wobei Ra und/oder Rb bevorzugt ausgewählt sind aus -C₁₃H₂₇ (F4), -C₁₅H₃₁ (F5), -C₁₇H₃₅ (F6), -C₁₉H₃₉ (F7) und den Resten F12, F13, F16, F17, F21 und F22.

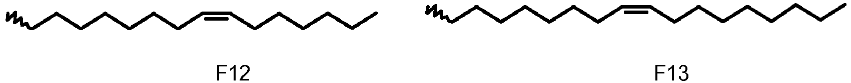

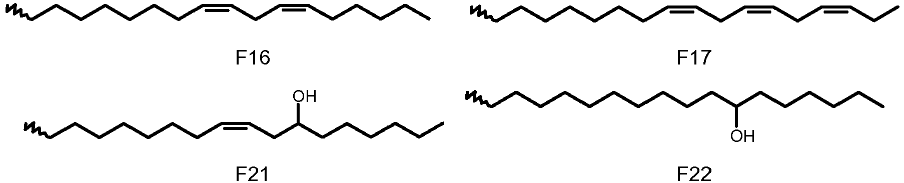

In einer Ausführungsform der vorliegenden Erfindung enthält das Mittel einen Fettsäurediester eines Alkandiols und zwei strukturgleichen Fettsäuren, wobei R1' und R3' beide für Wasserstoff stehen und n gleich 0 ist. Bevorzugt werden die Diester aus einem Alkandiol und zwei strukturgleichen Fettsäuren aus Verbindungen der Formel (II) ausgewählt, bei denen die Reste Ra und Rb stehen für

| Derivat der Formel (II) | Ra | Rb |
|---|---|---|
| 1 | F1 | F1 |
| 2 | F2 | F2 |
| 3 | F3 | F3 |
| 4 | F4 | F4 |
| 5 | F5 | F5 |
| 6 | F6 | F6 |
| 7 | F7 | F7 |
| 8 | F8 | F8 |
| 9 | F9 | F9 |
| 10 | F10 | F10 |
| 11 | F11 | F11 |
| 12 | F12 | F12 |
| 13 | F13 | F13 |
| 14 | F14 | F14 |
| 15 | F15 | F15 |
| 16 | F16 | F16 |
| 17 | F17 | F17 |
| 18 | F18 | F18 |
| 19 | F19 | F19 |
| 20 | F20 | F20 |
| 21 | F21 | F21 |
| 22 | F22 | F22 |

Färbungen mit besonders guter Waschechtheit und besonders hohem Glanz werden erhalten, wenn Ra und Rb beide für eine gesättigte unverzeigte, unsubstituierte C₁₄-C₂₀-Alkylgruppe stehen und wenn Ra und Rb gleich sind.

Eine weitere besonders bevorzugte Ausführungsform ist daher ein Mittel zum Färben und gegebenenfalls gleichzeitigen Aufhellen von keratinischen Fasern, welches dadurch gekennzeichnet ist, dass es als Fettsäurediester eines Alkandiols (b) eine Verbindung der Formel (II) enthält, bei der Ra und Rb beide für eine gesättigte unverzeigte, unsubstituierte C₁₄-C₂₀-Alkylgruppe stehen und Ra und Rb gleich sind.

In einer weiteren Ausführungsform der vorliegenden Erfindung enthält das erfindungsgemäße Mittel einen Fettsäurediester aus einem Alkandiol und zwei strukturell unterschiedlichen Fettsäuren, wobei R1' und R3' beide für Wasserstoff stehen und n gleich 0 ist. Die bevorzugten Verbindungen dieser Gruppe sind ausgewählt aus Verbindungen der Formel (II), bei denen die Reste Ra und Rb stehen für

| Derivat der Formel (II) | Ra | Rc |
|---|---|---|
| 23 | F4 | F5 |
| 24 | F4 | F6 |
| 25 | F4 | F7 |
| 26 | F4 | F12 |
| 27 | F4 | F13 |
| 28 | F4 | F16 |
| 29 | F4 | F17 |
| 30 | F4 | F21 |
| 31 | F4 | F22 |
| 32 | F5 | F4 |
| 33 | F5 | F6 |
| 34 | F5 | F7 |
| 35 | F5 | F12 |
| 36 | F5 | F13 |
| 37 | F5 | F16 |
| 38 | F5 | F17 |
| 39 | F5 | F21 |
| 40 | F5 | F22 |
| 41 | F6 | F4 |
| 42 | F6 | F5 |
| 43 | F6 | F7 |
| 44 | F6 | F12 |
| 45 | F6 | F13 |
| 46 | F6 | F16 |
| 47 | F6 | F17 |
| 48 | F6 | F21 |
| 49 | F6 | F22 |
| 50 | F7 | F4 |
| 51 | F7 | F5 |
| 52 | F7 | F6 |
| 53 | F7 | F12 |
| 54 | F7 | F13 |
| 55 | F7 | F16 |
| 56 | F7 | F17 |
| 57 | F7 | F21 |
| 58 | F7 | F22 |
| 59 | F12 | F4 |
| 60 | F12 | F5 |
| 61 | F12 | F6 |
| 62 | F12 | F7 |
| 63 | F12 | F13 |
| 64 | F12 | F16 |
| 65 | F12 | F17 |
| 66 | F12 | F21 |
| 67 | F12 | F22 |
| 68 | F13 | F4 |
| 69 | F13 | F5 |
| 70 | F13 | F6 |
| 71 | F13 | F7 |
| 72 | F13 | F12 |
| 73 | F13 | F16 |
| 74 | F13 | F17 |
| 75 | F13 | F21 |
| 76 | F13 | F22 |
| 77 | F16 | F4 |
| 78 | F16 | F5 |
| 79 | F16 | F6 |
| 80 | F16 | F7 |
| 81 | F16 | F12 |
| 82 | F16 | F13 |
| 83 | F16 | F17 |
| 84 | F16 | F21 |
| 85 | F16 | F22 |
| 86 | F17 | F4 |
| 87 | F17 | F5 |
| 88 | F17 | F6 |
| 89 | F17 | F7 |
| 90 | F17 | F12 |
| 91 | F17 | F13 |
| 92 | F17 | F16 |
| 93 | F17 | F21 |
| 94 | F17 | F22 |
| 95 | F21 | F4 |
| 96 | F21 | F5 |
| 97 | F21 | F6 |
| 98 | F21 | F7 |
| 99 | F21 | F12 |
| 100 | F21 | F13 |
| 101 | F21 | F16 |
| 102 | F21 | F17 |
| 103 | F21 | F22 |
| 104 | F22 | F4 |
| 105 | F22 | F5 |
| 106 | F22 | F6 |
| 107 | F22 | F7 |
| 108 | F22 | F12 |
| 109 | F22 | F13 |
| 110 | F22 | F16 |
| 111 | F22 | F17 |
| 112 | F22 | F21 |

Beispielsweise setzt sich die Struktur des Fettsäurediesters Nr. 112 aus Ethylenglykol und den Fettsäureresten Ra = F22 und Rb = F21 zusammen, woraus Struktur (II₁₁₂) resultiert. Die Strukturen aller weiteren Derivate lassen sich analog ableiten.

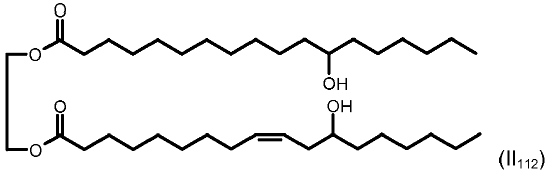

Insbesondere bevorzugt wird als Fettsäurediester eines Alkandiols (b) Ethylenglykoldistearat (INCI-Name: Ethylen Glycol Distearate) in den erfindungsgemäßen Mittel eingesetzt. Vertrieben wird diese Substanz beispielsweise unter den Handelsnamen Cutina AGS, Lipo EGDS, Loxiol 2723 und Eumulsan AGS.

Auch Propylengylkoldistearat (INCI-Name: Propylene glycol distearate, Diester von Stearinsäure mit 1,2-Propandiol) ist in diesem Zusammenhang ganz besonders bevorzugt.

Eine weitere besonders bevorzugte Ausführungsform ist daher ein Mittel zum Färben und gegebenenfalls gleichzeitigen Aufhellen von keratinischen Fasern, welches dadurch gekennzeichnet ist, dass es als Fettsäurediester eines Alkandiols (b) Ethylengykoldistearat und/oder Propylenglycoldistearat enthält.

Die erfindungsgemäßen Mittel zum Färben und/oder Aufhellen von keratinischen Fasern enthalten das bzw. die Fettsäurediester eines Alkandiols (b) bevorzugt in einem Anteil von 0,75 bis 3,0 Gew.-%, bezogen auf das Gesamtgewicht des Mittels. Hierbei sind die angegebenen Gewichtsanteile auf die Gesamtmenge aller im Mittel enthaltenen Komponenten (b) bezogen.

Eine weitere bevorzugte Ausführungsform ist daher ein Mittel zum Färben und gegebenenfalls gleichzeitigen Aufhellen von keratinischen Fasern, welches dadurch gekennzeichnet ist, dass es das oder die Fettsäurediester eines Alkandiols (b) in einem Anteil von 0,001 bis 15 Gew.-%, bevorzugt 0,05 bis 12 Gew.-%, besonders bevorzugt 0,1 bis 10,0 Gew.-%, insbesondere 0,5 bis 5,0 Gew.-%, und insbesondere bevorzugt von 0,75 bis 3,0 Gew.-%, jeweils bezogen auf das Gesamtgewicht des Mittels, enthält.

In einer weiteren bevorzugten Ausführungsform enthalten die erfindungsgemäßen Mittel neben dem Farbstoff der Formel (I) zusätzlich mindestens einen weiteren direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Die zusätzlichen direktziehenden Farbstoffe werden jeweils bevorzugt in einem Anteil von 0,001 bis 2 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17 sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)-amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochin-oxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Färbeergebnisse mit herausragender Farbintensität, Brillanz und guter Waschechtheit werden insbesondere dann erhalten, wenn die erfindungsgemäßen Mittel als weiteren direktziehenden Farbstoff mindestens einen Farbstoff ausgewählt aus D&C Red No. 33 (Acid Red 33), Acid Black No. 1, D&C Orange No. 4 (Acid Orange No. 4), Acid Red 18, Basic Red 76, Acid Violet 43, HC Blue No. 12, N-(2-Hydroxethyl)-4-methyl-2-nitroanilin (Methyl Yellow), HC Yellow No. 2, Red B 54 und 2-Amino-6-chlor-4-phenol enthalten.

Die erfindungsgemäßen Mittel können weiterhin auch als Oxidationsfärbemittel eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die direktziehenden Farbstoffe, Entwicklerkomponenten und Kupplerkomponenten werden bevorzugt jeweils in einer Menge von 0,0001 bis 5,0 Gew.-%, vorzugsweise 0,001 bis 2,5 Gew.-%, jeweils bezogen auf das anwendungsbereite Mittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Im Falle von Oxidationsfärbemitteln enthalten die Mittel bevorzugt ein Oxidationsmittel, bevorzugt Wasserstoffperoxid. Die Mengen an Wasserstoffperoxid entsprechen den Mengen in den erfindungsgemäßen Aufhellmitteln.

Die Färbemittel können weiterhin als aufhellende Färbemittel eingesetzt werden. Zur Erzielung des Aufhelleffekts enthalten die Mittel dazu Wasserstoffperoxid und/oder eines seiner festen Anlagerungsprodukte an organische oder anorganische Verbindungen.

Eine weitere Ausführungsform des ersten Erfindungsgegenstands ist daher dadurch gekennzeichnet, dass das Mittel zusätzlich Wasserstoffperoxid und/oder eines seiner festen Anlagerungsprodukte an organische oder anorganische Verbindungen enthält.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten.

Eine weitere bevorzugte Ausführungsform ist ein Mittel, zum Färben und gleichzeitigen Aufhellen keratinischer Fasern, welches zusätzlich Wasserstoffperoxid, eines seiner festen Anlagerungsprodukte an organische oder anorganische Verbindungen, Ammoniumperoxodisulfat, Kaliumperoxodisulfat und/oder Natriumperoxodisulfat jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, enthält.

Die anwendungsbereiten Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Erfindungsgemäß bevorzugte Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß bevorzugte Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid® S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten.

Die anwendungsbereiten Färbemittel können weitere Hilfs- und Zusatzstoffe enthalten. So hat es sich als vorteilhaft erwiesen, wenn die Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind
- anionische Polymere,
- kationische, synthetische Polymere;
- natürlich vorkommende Verdickungsmittel, wie nichtionische Guargums, Skleroglucangums oder Gummi arabicum, Ghatti-Gummi, Karaya-Gummi, Tragant-Gummi, Carrageen-Gummi, Johannisbrotkernmehl, Pektine, Stärke-Fraktionen und Derivate wie Amylose, Amylopektin und Dextrine, sowie nichtionische Cellulosederivate, wie beispielsweise Methylcelluloseund Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Der pH-Wert der erfindungsgemäßen Mittel kann daher zwischen 3 und 11 liegen. Es ist bevorzugt, wenn der pH-Wert des anwendungsbereiten Mittels zwischen 5 und 11, insbesondere zwischen 5 und 8, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; Strukturanten wie Glucose, Maleinsäure und Milchsäure, haarkonditionierende Verbindungen wie Phospholipide, beispielsweise Lecitin und Kephaline.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Zur Anwendung der erfindungsgemäßen Mittel eignet sich insbesondere ein Verfahren zum Färben von keratinischen Fasern, insbesondere menschlichen Haaren, welches dadurch gekennzeichnet ist, dass ein Mittel des ersten Erfindungsgegenstands auf die keratinhaltigen Fasern aufgebracht, 5 bis 60 Minuten auf der Faser belassen und anschließend mit Wasser wieder ausgespült oder mit einem Shampoo ausgewaschen wird. Bevorzugt beträgt die Einwirkzeit der anwendungsbereiten Färbemittel 5 bis 45 min, insbesondere 10 bis 40 min, besonders bevorzugt 15 bis 35 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Aufhellmittel einen stark tensidhaltigen Träger besitzt.

Die erfindungsgemäßen Mittel können als Einkomponentenmittel (Färbe- und Aufhellmittel) oder als Mehrkomponentenmittel wie Zweikomponenten Mittel oder Dreikomponentenmittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Enthält das erfindungsgemäße Mittel sowohl direktziehende Farbstoffe - sowie gegebenenfalls zusätzlich Oxidationsfarbstoffvorprodukte - als auch Oxidationsmittel, so werden diese, um eine vorzeitige, unerwünschte Reaktion miteinander zu verhindern, zweckmäßigerweise getrennt voneinander konfektioniert und erst unmittelbar vor der Anwendung in Kontakt gebracht.

Ein Färbe- und Aufhellverfahren, bei dem die Färbecreme und das Oxidationsmittel zunächst getrennt vorliegen, ist daher bevorzugt. Ein weiterer Gegenstand der vorliegenden Erfindung ist daher ein Verfahren zum Färben und Aufhellen von menschlichen Haaren, bei dem eine Zusammensetzung auf wässriger Grundlage, enthaltend Wasserstoffperoxid, mit einem erfindungsgemäßen Mittel enthaltend mindestens eine Verbindung der Formel (I) zu einer homogenen Zusammensetzung vermischt, und diese auf das Haar aufgebracht wird. Der Difettsäureester eines Alkanols (b) kann in diesem Fall sowohl zusammen mit der Wasserstoffperoxid-Lösung als auch mit der Verbindung der Formel (I) konfektioniert werden.

In einer weiteren Ausführungsform der vorliegenden Erfindung sind daher Mittel bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden, und wobei ein Container ein Mittel (A), welches in einem kosmetischen Träger mindestens einen kationischen Anthrachinonfarbstoff der Formel (I) - sowie gegebenenfalls zusätzlich Oxidationsfarbstoffvorprodukte enthält, und ein weiterer Container eine Oxidationsmittelzubereitung (B), enthaltend mindestens ein Oxidationsmittel, enthält. Der oder die Difettsäureester eines Alkandiols (b) können hierbei sowohl zusammen mit dem kationischen Anthrachinonfarbstoff der Formel (I) in Container (A) als auch zusammen mit der Oxidationsmittelzubereitung in Container (B) konfektioniert werden.

Die Formulierung einer Kombination von (a) Verbindungen der allgemeinen Formel (I) mit mindestens einem (b) Difettsäureester eines Alkandiols eignet sich hervorragend zur Erzeugung von intensiven Färbungen mit hoher Brillanz, hohem Glanz, einer niedrigen Selektivität in Verbindung mit einer hervorragenden Waschechtheit. Die Mittel sind ebenfalls hervorragend geeignet, das Austrocknen der Kopfhaut zu minimieren oder zu verhindern.

Ein weiterer Gegenstand der vorliegenden Erfindung ist die Verwendung eines Mittels des ersten Erfindungsgegenstands zur Erzeugung von Haarfärbungen mit erhöhtem Glanz, intensivem Farbergebnis mit verbesserten Echtheitseigenschaften und/oder verringerter Selektivität.

Bezüglich weiterer bevorzugter Ausführungsformen der erfindungsgemäßen Verfahren und Verwendung gilt mutatis mutandis das zu den erfindungsgemäßen Mitteln Gesagte.

### Ausführungsbeispiele

Es wurden die folgenden Rezepturen hergestellt. Die Mengenangaben verstehen sich, sofern nichts anderes vermerkt ist, jeweils in Gewichtsprozent.

### Formulierungsbeispiel 1

| **Beschreibung** | **Gew.-%** |
|---|---|
| Polyquaternium-10 | 0,45 |
| Citric acid Monohydrate | 0,70 |
| Timiron Super Silver | 0,20 |
| Cationic Blue 347 | 0,20 |
| Salicylic acid | 0,20 |
| Disodium Cocoamphodiacetate | 7,00 |
| Na-benzoate | 0,50 |
| Nicotinamide | 0,50 |
| Sodium Pyrrolidinon-2-carboxylate | 2,00 |
| Cutina HR | 1,00 |
| PEG-7 Glyceryl Cocoate | 0,50 |
| Sodium Myreth Sulfate (2 EO), 70% | 24,00 |
| NaOH, 50 % | 0,15 |
| D-Panthenol, 75 % | 0,50 |
| Euperlan PK 3000 AM | 2,60 |
| ProSina | 2,0 |
| Sericin H | 0,20 |
| Caramel syrup, 75 % | 0,60 |
| Aprikosenkernöl | 0,10 |
| PEG-40 hydrogenated Castor Oil | 0,60 |
| Natriumchlorid | 0,20 |
| Antil 141 L | 1,00 |
| Hydrolyzed Silk Protein | 1,50 |
| Benzophenone-4 | 0,50 |
| Parfum | qs |
| Wasser | Ad 100 |

### Rezepturbestandteile

| | |
|---|---|
| Cationic Blue 347 | 3-[(4-Amino-9,10-dihydro-3-methyl-9,10-dioxo-1-anthracenyl)amino]-N,N,N-trimethyl-1-propanaminiummethylsulfat, |
| Timiron Super Silver | INCI-Bezeichnung: Mica, Titanium Dioxide (Merck KGaA) |
| Cutina HR | INCI-Bezeichnung: Hydrogenated Castor Oil (BASF) |
| Euperlan PK 3000 AM | ca. 43% Festsubstanz; INCI-Bezeichnung: Aqua, Ethylene Glycol Distearate, Glycerin, Laureth-4, Cocamidopropyl Betaine, Formic Acid (BASF) |
| ProSina | INCI-Bezeichnung: Aqua, hydrolyzed Keratin (Keratec/Croda) |
| Sericin H | INCI-Bezeichnung: Sericin (Pentapharm) |
| Antil 141 L | ca. 40% Aktivsubstanz; INCI-Bezeichnung: Propylene Glycol, PEG-55 Propylene Glycol Oleate (Goldschmidt/Evonik) |

Die Färbeformulierungen wurden auf Haarsträhnen aufgetragen und dort für 30 Minuten bei Raumtemperatur belassen. Anschließend wurden die Fasern gründlich mit Wasser ausgespült und getrocknet.

Die behandelten Fasern zeichneten sich durch intensive Färbungen mit hohem Glanz, einer hervorragenden Waschechtheit und weichen Griff aus.

## Patentansprüche

1. Mittel zum Färben und gegebenenfalls gleichzeitigen Aufhellen von keratinischen Fasern, insbesondere menschlichen Haaren, enthaltend in einem kosmetischen Träger
(a) mindestens eine Verbindung der Formel (I) 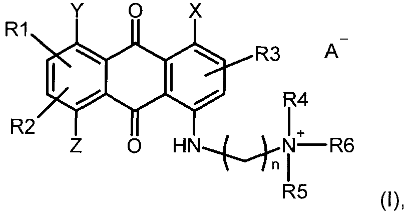 worin
R1, R2, R3 jeweils unabhängig voneinander für Wasserstoff, Halogen, eine Nitrogruppe, eine Cyanogruppe, eine Carboxylgruppe, eine Sulfonsäuregruppe, eine Hydroxygruppe, eine C₁-C₆-Acylaminogruppe, eine Carboxamidgruppe, eine Sulfonamidgruppe, eine C₁-C₆-Alkylgruppe, C₁-C₆-Alkoxygruppe, eine C₂-C₆-Alkenylgruppe, eine C₂-C₆-Hydroxyalkylgruppe oder eine C₁-C₆-Alkoxy-C₂-C₆-alkylgruppe stehen;
R4, R5, R6 jeweils unabhängig voneinander für eine C₁-C₆-Alkylgruppe, eine C₂-C₆-Alkenylgruppe, für eine C₂-C₆-Hydroxyalkylgruppe oder eine C₁-C₆-Alkoxy-C₂-C₆-alkylgruppe stehen;
oder R4 und R5 bilden zusammen mit dem quartären Stickstoffatom, an das sie gebunden sind, einen 5-, 6- oder 7-gliedrigen Ring, der gegebenenfalls weitere Heteroatome enthalten kann und/oder weitere Substituenten tragen kann;
X, Y, Z jeweils unabhängig voneinander für Wasserstoff, eine Hydroxygruppe oder eine Gruppe N(R7)(R8) stehen, worin
R7 und R8 jeweils unabhängig voneinander für Wasserstoff, eine C₁-C₆-Alkylgruppe, eine C₂-C₆-Alkenylgruppe, für eine C₂-C₆-Hydroxyalkylgruppe oder eine C₁-C₆-Alkoxy-C₂-C₆-alkylgruppe stehen,
unter der Maßgabe, dass mindestens einer der Reste X, Y, Z für eine Gruppe N(R7)(R8) steht,
n für eine Zahl von 2 bis 6 steht,
A⁻ für ein physiologisch verträgliches Anion steht,
und
(b) mindestens einen Fettsäurediester eines Alkandiols,
wobei das Mittel den oder die Fettsäurediester eines Alkandiols (b) in einem Anteil von 0.5 bis 5,0 Gew.-%, jeweils bezogen auf das Gesamtgewicht des Mittels, enthält.

2. Mittel nach Anspruch 1, **dadurch gekennzeichnet, dass** es eine Verbindung der Formel (I) enthält, worin wenigstens einer der Reste R1, R2 und/oder R3 für eine C₁-C₆-Alkylgruppe steht.

3. Mittel nach Anspruch 1 oder 2, **dadurch gekennzeichnet, dass** es eine Verbindung der Formel (I) enthält, worin mindestens X für eine Gruppe NH₂ steht.

4. Mittel nach einem der Ansprüche 1 bis 3, **dadurch gekennzeichnet, dass** es als Verbindung der Formel (I) die Verbindung gemäß Formel (la) enthält, 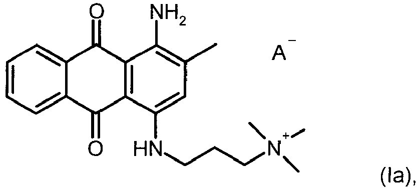 worin A⁻ für ein physiologisch verträgliches Anion, bevorzugt für Methylsulfat (H₃COSO₃⁻), steht.

5. Mittel nach einem der Ansprüche 1 bis 4, **dadurch gekennzeichnet, dass** es die Verbindung(en) gemäß Formel (I) in einem Anteil von 0,0001 bis 5 Gew.-%, bevorzugt 0,005 bis 3,5 Gew.-%, besonders bevorzugt 0,01 bis 2,5 Gew.-%, insbesondere 0,05 bis 1,5 Gew.-%, und insbesondere bevorzugt von 0,01 bis 1,0 Gew.-%, jeweils bezogen auf das Gesamtgewicht des Mittels, enthält.

6. Mittel nach einem der Ansprüche 1 bis 5, **dadurch gekennzeichnet, dass** es als Fettsäurediester eines Alkandiols (b) eine Verbindung der Formel (II) enthält, 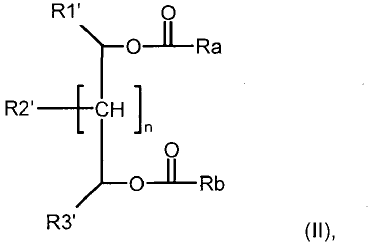 worin
R1', R3' unabhängig voneinander für Wasserstoff, eine C₁-C₈-Alkylgruppe, eine Arylgruppe oder eine Hydroxy-C₁-C₆-alkylgruppe stehen,
R2' für Wasserstoff, eine Hydroxygruppe, eine C₁-C₈-Alkylgruppe, eine Arylgruppe oder eine Hydroxy-C₁-C₆-alkylgruppe steht,
n für die Zahlen 0 bis 8 steht und
Ra, Rb unabhängig voneinander für eine gesättigte oder ungesättigte, unverzweigte oder verzeigte, unsubstituierte oder substituierte C₇-C₂₃-Alkylgruppe stehen, wobei Ra und/oder Rb bevorzugt ausgewählt sind aus -C₁₃H₂₇ (F4), -C₁₅H₃₁ (F5), -C₁₇H₃₅(F6), -C₁₉H₃₉(F7) und den Resten F12, F13, F16, F17, F21 und F22.

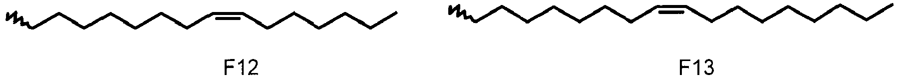

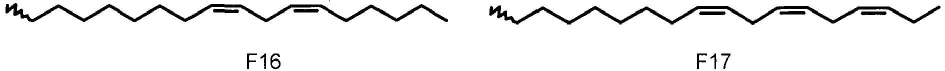

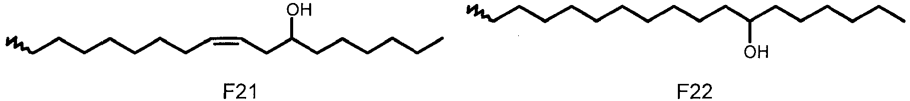

7. Mittel nach einem der Ansprüche 1 bis 6, **dadurch gekennzeichnet, dass** es als Fettsäurediester eines Alkandiols (b) eine Verbindung der Formel (II) enthält, bei der Ra und Rb beide für eine gesättigte unverzweigte, unsubstituierte C₁₄-C₂₀-Alkylgruppe stehen und Ra und Rb gleich sind.

8. Mittel nach einem der Ansprüche 1 bis 7, **dadurch gekennzeichnet, dass** es als Fettsäurediester eines Alkandiols (b) Ethylengykoldistearat und/oder Propylenglycoldistearat enthält.

9. Mittel nach einem der Ansprüche 1 bis 8, **dadurch gekennzeichnet ist, dass** es den oder die Fettsäurediester eines Alkandiols (b) in einem Anteil von 0,75 bis 3,0 Gew.-%, jeweils bezogen auf das Gesamtgewicht des Mittels, enthält.

10. Mittel nach einem der Ansprüche 1 bis 9, **dadurch gekennzeichnet, dass** es zusätzlich Wasserstoffperoxid und/oder eines seiner festen Anlagerungsprodukte an organische oder anorganische Verbindungen enthält.

11. Mittel nach einem der Ansprüche 1 bis 10, **dadurch gekennzeichnet, dass** es zusätzlich mindestens ein anionisches Tensid enthält.

12. Mittel nach einem der Ansprüche 1 bis 11, **dadurch gekennzeichnet, dass** es zusätzlich mindestens ein zwitterionisches Tensid enthält.

13. Mittel nach einem der Ansprüche 1 bis 12, **dadurch gekennzeichnet, dass** es zusätzlich mindestens ein amphoteres Tensid enthält.

14. Verwendung eines Mittels nach einem der Ansprüche 1 bis 13 zur Erzeugung von Haarfärbungen mit verringerter Selektivität.

## Claims

1. An agent for coloring and optionally simultaneously lightening keratin fibers, in particular human hair, containing, in a cosmetic carrier
(a) at least one compound of formula (I) 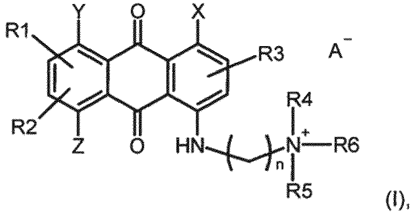 where
R1, R2, R3 each represent, independently of one another, hydrogen, halogen, a nitro group, a cyano group, a carboxyl group, a sulfonic acid group, a hydroxy group, a C₁-C₆ acyl amino group, a carboxamide group, a sulfonamide group, a C₁-C₆ alkyl group, C₁-C₆ alkoxy group, a C₂-C₆ alkenyl group, a C₂-C₆ hydroxyalkyl group or a C₁-C₆ alkoxy-C₂-C₆ alkyl group;
R4, R5, R6 each represent, independently of one another, a C₁-C₆ alkyl group, a C₂-C₆ alkenyl group, a C₂-C₆ hydroxyalkyl group or a C₁-C₆ alkoxy-C₂-C₆ alkyl group;
or R4 and R5, together with the quaternary nitrogen atom to which they are bound, form a 5-, 6- or 7-membered ring, which can optionally contain additional heteroatoms and/or can carry additional substituents;
X, Y, Z each represent, independently of one another, hydrogen, a hydroxy group or an N(R7)(R8) group,
where
R7 and R8 each represent, independently of one another, hydrogen, a C₁-C₆ alkyl group, a C₂-C₆ alkenyl group, a C₂-C₆ hydroxyalkyl group or a C₁-C₆ alkoxy-C₂-C₆ alkyl group, with the proviso that at least one of the functional groups X, Y, Z represents an N(R7)(R8) group,
n represents a number from 2 to 6,
A⁻ represents a physiologically acceptable anion,
and
(b) at least one fatty acid diester of an alkane diol, wherein the agent contains the fatty acid diester(s) of an alkane diol (b) in a proportion of from 0.5 to 5.0 wt.%, in each case based on the total weight of the agent.

2. The agent according to claim 1, **characterized in that** it contains a compound of formula (I), where at least one of the functional groups R1, R2 and/or R3 represents a C₁-C₆ alkyl group.

3. The agent according to claim 1 or 2, **characterized in that** it contains a compound of formula (I), where at least X represents an NH₂ group.

4. The agent according to one of claims 1 to 3, **characterized in that** it contains the compound according to formula (Ia) as the compound of formula (I), 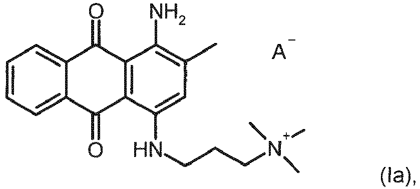 where A⁻ represents a physiologically acceptable anion, preferably methyl sulfate (H₃COSO₃⁻).

5. The agent according to one of claims 1 to 4, **characterized in that** it contains the compound(s) according to formula (I) in a proportion of from 0.0001 to 5 wt.%, preferably from 0.005 to 3.5 wt.%, particularly preferably from 0.01 to 2.5 wt.%, in particular from 0.05 to 1.5 wt.%, and particularly preferably from 0.01 to 1.0 wt.%, in each case based on the total weight of the agent.

6. The agent according to one of claims 1 to 5, **characterized in that** it contains a compound of formula (II) as a fatty acid diester of an alkane diol (b), 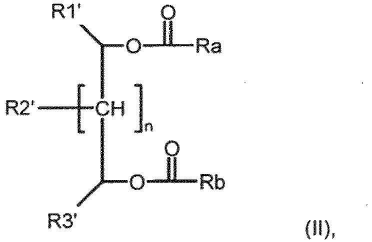 where
R1', R3' represent, independently of one another, hydrogen, a C₁-C₈ alkyl group, an aryl group or a hydroxy-C₁-C₆ alkyl group,
R2' represents hydrogen, a hydroxy group, a C₁-C₈ alkyl group, an aryl group or a hydroxy-C₁-C₆ alkyl group,
n represents the numbers 0 to 8, and
Ra, Rb represent, independently of one another, a saturated or unsaturated, unbranched or branched, unsubstituted or substituted C₇-C₂₃ alkyl group, Ra and/or Rb preferably being selected from -C₁₃H₂₇ (F4), -C₁₅H₃₁ (F5),-C₁₇H₃₅ (F6), -C₁₉H₃₉ (F7) and the functional groups F12, F13, F16, F17, F21 and F22.

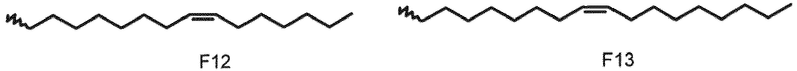

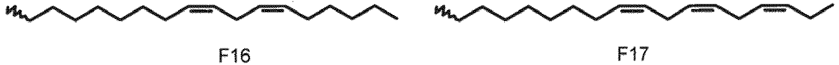

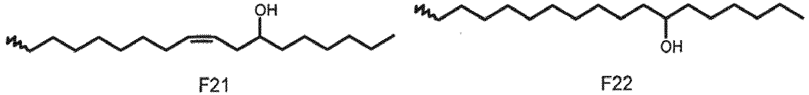

7. The agent according to one of claims 1 to 6, **characterized in that** it contains a compound of formula (II) as a fatty acid diester of an alkane diol (b), in which Ra and Rb both represent a saturated, unbranched, unsubstituted C₁₄-C₂₀ alkyl group and Ra and Rb are the same.

8. The agent according to one of claims 1 to 7, **characterized in that** it contains ethylene glycol distearate and/or propylene glycol distearate as a fatty acid diester of an alkane diol (b).

9. The agent according to one of claims 1 to 8, **characterized in that** it contains the fatty acid diester(s) of an alkane diol (b) in a proportion of from 0.75 to 3.0 wt.%, in each case based on the total weight of the agent.

10. The agent according to one of claims 1 to 9, **characterized in that** it additionally contains hydrogen peroxide and/or one of the solid addition products thereof to organic or inorganic compounds.

11. The agent according to one of claims 1 to 10, **characterized in that** it additionally contains at least one anionic surfactant.

12. The agent according to one of claims 1 to 11, **characterized in that** it additionally contains at least one zwitterionic surfactant.

13. The agent according to one of claims 1 to 12, **characterized in that** it additionally contains at least one amphoteric surfactant.

14. The use of an agent according to one of claims 1 to 13 for producing hair dyes having reduced selectivity.

## Revendications

1. Agent de coloration et le cas échéant d'éclaircissement simultané de fibres kératiniques, en particulier de cheveux humains, contenant dans un support cosmétique
(a) au moins un composé de la formule (I) 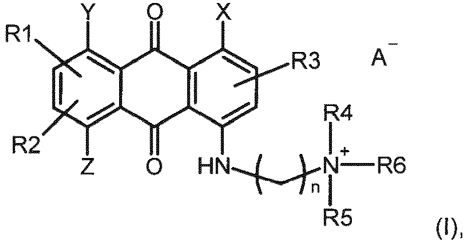 dans laquelle
R1, R2, R3 représentent respectivement indépendamment l'un de l'autre de l'hydrogène, de l'halogène, un groupe nitro, un groupe cyano, un groupe carboxyle, un groupe d'acide sulfonique, un groupe hydroxy, un groupe acylamino en C₁-C₆, un groupe carboxamide, un groupe sulfamidé, un groupe alkyle en C₁-C₆, un groupe alkoxy en C₁-C₆, un groupe alcényle en C₂-C₆, un groupe hydroxyalkyle en C₂-C₆ ou un groupe alkyle en C₂-C₆ alcoxy en C₁-C₆ ;
R4, R5, R6 représentent respectivement indépendamment l'un de l'autre un groupe alkyle en C₁-C₆, un groupe alcényle en C₂-C₆, un groupe hydroxyalkyle en C₂-C₆ ou un groupe alkyle en C₂-C₆ alcoxy en C₁-C₆ ;
ou R4 et R5 forment, ensemble avec l'atome d'azote quaternaire auquel ils sont liés, un cycle à 5, 6, ou 7 chaînons, qui peut le cas échéant contenir d'autres hétéroatomes et/ou peut porter d'autres substituts ;
X, Y, Z représentent respectivement indépendamment l'un de l'autre de l'hydrogène, un groupe hydroxy ou un groupe N(R7)(R8),
dans lesquels
R7 et R8 représentent respectivement indépendamment l'un de l'autre de l'hydrogène, un groupe alkyle en C₁-C₆, un groupe alcényle en C₂-C₆, un groupe hydroxyalkyle en C₂-C₆ ou un groupe alkyle en C₂-C₆ alcoxy en C₁-C₆, à la condition qu'au moins un des résidus X, Y, Z représente un groupe N(R7)(R8),
n représente un chiffre allant de 2 à 6,
A⁻ représente un anion compatible physiologiquement,
et
(b) au moins un diester d'acide gras d'un alcanediol, l'agent contenant le ou les diester(s) d'acide gras d'un alcanediol (b) dans une proportion allant de 0,5 à 5,0 % en poids, respectivement rapporté au poids total de l'agent.

2. Agent selon la revendication 1, **caractérisé en ce qu'**il contient un composé de la formule (I), dans laquelle au moins un des résidus R1, R2 et/ou R3 représente un groupe alkyle en C₁-C₆.

3. Agent selon la revendication 1 ou 2, **caractérisé en ce qu'**il contient un composé de la formule (I), dans laquelle au moins X représente un groupe NH₂.

4. Agent selon l'une des revendications 1 à 3, **caractérisé en ce qu'**il contient, en tant que composé de la formule (I), le composé selon la formule (la), 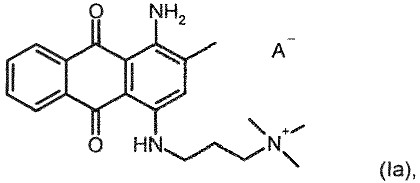 dans laquelle A⁻ représente un anion compatible physiologiquement, de préférence du méthylsulfate (H₃COSO₃⁻).

5. Agent selon l'une des revendications 1 à 4, **caractérisé en ce qu'**il contient le(s) composé(s) selon la formule (I) dans une proportion allant de 0,0001 à 5 % en poids, de préférence de 0,005 à 3,5 % en poids, de manière particulièrement préférée de 0,01 à 2,5 % en poids, en particulier de 0,05 à 1,5 % en poids, et de manière tout particulièrement préférée de 0,01 à 1,0 % en poids, respectivement rapporté au poids total de l'agent.

6. Agent selon l'une des revendications 1 à 5, **caractérisé en ce qu'**il contient, en tant que diester d'acide gras d'un alcanediol (b), un composé de la formule (II), 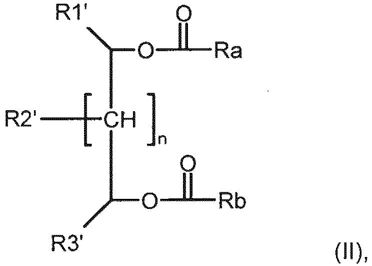 dans laquelle
R1', R3' représentent indépendamment l'un de l'autre de l'hydrogène, un groupe alkyle en C₁-C₈, un groupe aryle ou un groupe hydroxyalkyle en C₁-C₆,
R2' représente de l'hydrogène, un groupe hydroxy, un groupe alkyle en C₁-C₈, un groupe aryle ou un groupe hydroxyalkyle en C₁-C₆,
n représente les chiffres 0 à 8 et
Ra, Rb représentent indépendamment l'un de l'autre un groupe alkyle en C₇-C₂₃ saturé ou insaturé, non-ramifié ou ramifié, non-substitué ou substitué, Ra et/ou Rb étant de préférence sélectionnés parmi -C₁₃H₂₇ (F4), -C₁₅H₃₁ (F5),-C₁₇H₃₅(F6), -C₁₉H₃₉(F7) et les résidus F12, F13, F16, F17, F21 et F22.

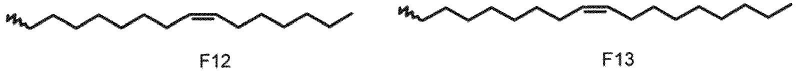

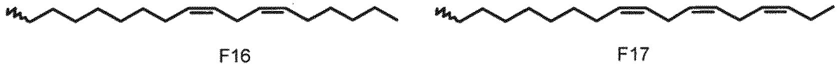

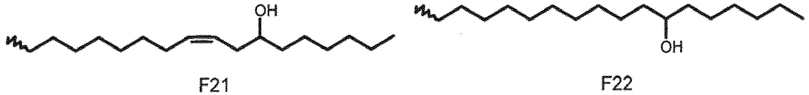

7. Agent selon l'une des revendications 1 à 6, **caractérisé en ce qu'**il contient, en tant que diester d'acide gras d'un alcanediol (b), un composé de la formule (II), pour laquelle Ra et Rb représentent tous deux un groupe alkyle en C₁₄-C₂₀ saturé, non-ramifié, non-substitué et Ra et Rb sont identiques.

8. Agent selon l'une des revendications 1 à 7, **caractérisé en ce qu'**il contient, en tant que diester d'acide gras d'un alcanediol (b), du distéarate d'éthylène glycol et/ou du distéarate de propylène glycol.

9. Agent selon l'une des revendications 1 à 8, **caractérisé en ce qu'**il contient le ou les diester(s) d'acide gras d'un alcanediol (b) dans une proportion allant de 0,75 à 3,0 % en poids, respectivement rapporté au poids total de l'agent.

10. Agent selon l'une des revendications 1 à 9, **caractérisé en ce qu'**il contient de plus du peroxyde d'hydrogène et/ou un de ses produits d'addition solides sur des composés organiques ou inorganiques.

11. Agent selon l'une des revendications 1 à 10, **caractérisé en ce qu'**il contient de plus au moins un tensioactif anionique.

12. Agent selon l'une des revendications 1 à 11, **caractérisé en ce qu'**il contient de plus au moins un tensioactif zwitterionique.

13. Agent selon l'une des revendications 1 à 12, **caractérisé en ce qu'**il contient de plus au moins un tensioactif amphotère.

14. Utilisation d'un agent selon l'une des revendications 1 à 13 pour créer des colorations capillaires avec une sélectivité réduite.
